# EUROPEAN PATENT APPLICATION

(11) **EP 4 134 816 A1**
(43) Date of publication of application: **15.02.2023**
(21) Application number: 22175919.4
(22) Date of filing: 27.05.2022
(51) Int. Cl.: G06F 8/65, G06F 3/01, G06F 9/44, G06Q 10/00

(54) **AUTO-IMPROVING SOFTWARE SYSTEM FOR USER BEHAVIOR MODIFICATION**

(30) Priority: 09.08.2021 US 202117397801
(71) Applicant: Intuit Inc., Mountain View, CA 94043 (US)
(72) Inventor: DAVID, Daniel Ben, Mountain View, California 94043 (US); MUKHERJEE, Saikat, Mountain View, California 94043 (US); RANGANATHAN, Nirmala, Mountain View, California 94043 (US); HORESH, Yair, Mountain View, California 94043 (US)
(74) Representative: D Young & Co LLP

(57) **Abstract**

A method including generating, by a state engine from data describing behaviors of users in an environment external to the state engine, an executable process. An agent executes the executable process by determining, from the data describing the behaviors of the users, a problem of at least some of the users, and selects, based on the problem, a chosen action to alter the problem. At a first time, a first electronic communication describing the chosen action to the at least some of the users is transmitted. Ongoing data describing ongoing behaviors of the users is monitored. A reward is generated based on the ongoing data to change a parameter of the agent. The parameter of the agent is changed to generate a modified agent. The modified agent executes the executable process to select a modified action. At a second time, a second electronic communication describing the modified action is transmitted.

## Description

### BACKGROUND

Software updates are generated manually. Feedback regarding operation of the software is provided to a computer programmer. Based on the feedback, the computer programmer codes new or modified functionality for the software to generate the software updates. The process of generating software updates manually is time and labor intensive.

### SUMMARY

Particular aspects are set out in the appended independent claims. Various optional embodiments are set out in the dependent claims.

The one or more embodiments provide for a method. The method includes generating, by a state engine from data describing behaviors of users operating in a computer environment external to the state engine, an executable process. The method also includes executing, by an agent, the executable process, by determining, from the data describing the behaviors of the users, a problem of at least some of the users, and selecting, based on the problem, a chosen action to alter the problem. The method also includes transmitting, at a first time, a first electronic communication describing the chosen action to the at least some of the users. The method also includes monitoring ongoing data describing ongoing behaviors of the users. The method also includes generating, based on the ongoing data, a reward. The reward is configured to change a parameter of the agent. The method also includes changing the parameter of the agent to generate a modified agent. The method also includes executing, by the modified agent, the executable process to select a modified action. The method also includes transmitting, at a second time, a second electronic communication describing the modified action.

The one or more embodiments also provide for a system. The system includes a processor and a data repository in communication with the processor. The data repository stores data describing behaviors of users operating in a computer environment. The data repository also stores a problem of at least some of the users, and actions. The data repository also stores a chosen action, from the actions, to alter the problem. The data repository also stores a modified action, from the actions. The data repository also stores a first electronic communication describing the chosen action. The data repository also stores a second electronic communication describing the modified action. The data repository also stores ongoing data, describing ongoing behaviors of the users. The data repository also stores a reward configured to change a parameter. The data repository also stores an executable process. The system also includes a state engine executable by the processor to generate, from the data, an executable process. The computer environment is external to the state engine. The system also includes an agent executable by the processor to execute the process by determining, from the data describing the behaviors of the users, a problem of at least some of the users, and selecting, based on the problem, a chosen action to alter the problem. The agent is also executable by the processor to transmit, at a first time, the first electronic communication to at least some of the users. The agent is also executable by the processor to monitor the ongoing data. The agent is also executable by the processor to generate the reward configured to change the parameter. The parameter is associated with the agent. The agent is also executable by the processor to modify the agent to generate a modified agent. The agent is also executable by the processor to execute, by the modified agent, the executable process to select the modified action. The agent is also executable by the processor to transmit, at a second time, the second electronic communication.

The one or more embodiments also provide for a computer readable medium comprising computer readable program code which, when executed by a processor, implements a computer-implemented method. The computer-implemented method includes generating, by a state engine from data describing behaviors of users operating in a computer environment external to the state engine, an executable process. The computer-implemented method also includes executing, by an agent, the executable process, by determining, from the data describing the behaviors of the users, a problem of at least some of the users, and selecting, based on the problem, a chosen action to alter the problem. The computer-implemented method also includes transmitting, at a first time, a first electronic communication describing the chosen action to the at least some of the users. The computer-implemented method also includes monitoring ongoing data describing ongoing behaviors of the users. The computer-implemented method also includes generating, based on the ongoing data, a reward. The reward is configured to change a parameter of the agent. The computer-implemented method also includes changing the parameter of the agent to generate a modified agent. The computer-implemented method also includes executing, by the modified agent, the executable process to select a modified action. The computer-implemented method also includes transmitting, at a second time, a second electronic communication describing the modified action.

Other aspects of the present teachings will be apparent from the following description and the appended claims.

### BRIEF DESCRIPTION OF DRAWINGS

FIG. 1 shows a computing system for automatically updating software, in accordance with one or more embodiments.
FIG. 2A and FIG. 2B are flowcharts of a method for automatically updating software, in accordance with one or more embodiments.
FIG. 3 shows an example of automatically updating software according to the methods and devices described with respect to FIG. 1, FIG. 2A, and FIG. 2B, in accordance with one or more embodiments.
FIG. 4A and FIG. 4B shows a computing system, in accordance with one or more embodiments.

### DETAILED DESCRIPTION

Specific embodiments will now be described in detail with reference to the accompanying figures. Like elements in the various figures are denoted by like reference numerals for consistency.

In the following detailed description of embodiments, numerous specific details are set forth in order to provide a more thorough understanding of the present teachings. However, it will be apparent to one of ordinary skill in the art that the approaches set out herein may be practiced without these specific details. In other instances, well-known features have not been described in detail to avoid unnecessarily complicating the description.

Throughout the application, ordinal numbers (e.g., first, second, third, etc.) may be used as an adjective for an element (i.e., any noun in the application). The use of ordinal numbers is not to imply or create any particular ordering of the elements nor to limit any element to being only a single element unless expressly disclosed, such as by the use of the terms "before", "after", "single", and other such terminology. Rather, the use of ordinal numbers is to distinguish between the elements. By way of an example, a first element is distinct from a second element, and the first element may encompass more than one element and succeed (or precede) the second element in an ordering of elements.

The term "about," when used with respect to a computer or a computer-executed instruction, refers to a computer engineering tolerance anticipated or determined by a computer scientist or computer technician of ordinary skill in the art. The exact quantified degree of an engineering tolerance depends on the software and/or hardware in use and the technical property being measured. For a non-limiting example, two processes may be "about" concurrent when one process is executed within a pre-defined number of processor operations of the other process. In another non-limiting example in which an algorithm compares a first property to a second property, the first property may be "about" equal to the second property when the two properties are within a pre-determined range of measurement. Engineering tolerances could be loosened in other embodiments; i.e., outside of the above-mentioned pre-determined range in one embodiment, but inside another pre-determined range in another embodiment. In any case, the ordinary artisan is capable of assessing what is an acceptable engineering tolerance for a particular algorithm, process, or hardware arrangement, and thus is capable of assessing how to determine the variance of measurement contemplated by the term "about."

As used herein, the term "connected to" contemplates multiple meanings. A connection may be direct or indirect. For example, computer A may be directly connected to computer B by means of a direct communication link. Computer A may be indirectly connected to computer B by means of a common network environment to which both computers are connected. A connection may be wired or wireless. A connection may be temporary, permanent, or semi-permanent communication channel between two entities.

As used herein, an entity is an electronic device, not necessarily limited to a computer. Thus, an entity may be a mobile phone, a smart watch, a laptop computer, a desktop computer, a server computer, *etc.* As used herein, the term "computer" is synonymous with the word "entity," unless stated otherwise.

In general, the one or more embodiments are directed to the automated improvement of software. In particular, the one or more embodiments relate to the automated improvement of software programmed to monitor electronic data describing human behavior, and to transmit suggestions for alternative behavior to the computers of the human users. In particular, the one or more embodiments monitor measured changes in human behavior, as determined using measurable computer-related parameters. The measured changes are used to generate a feedback system. The feedback system is used to change the execution of a state function by a machine learning model, referred-to as an agent. In some embodiments, the state function also may be changed by changing the output of a state engine which supplies the function to the agent.

In either or both cases, the software *(i.e.* the agent) is automatically updated over time. The automatically updated software generates improved output, in that the messages to the computers of the human users are correlated to improvements to desired human behaviors, as measured by ongoing data received from the computers of the human users.

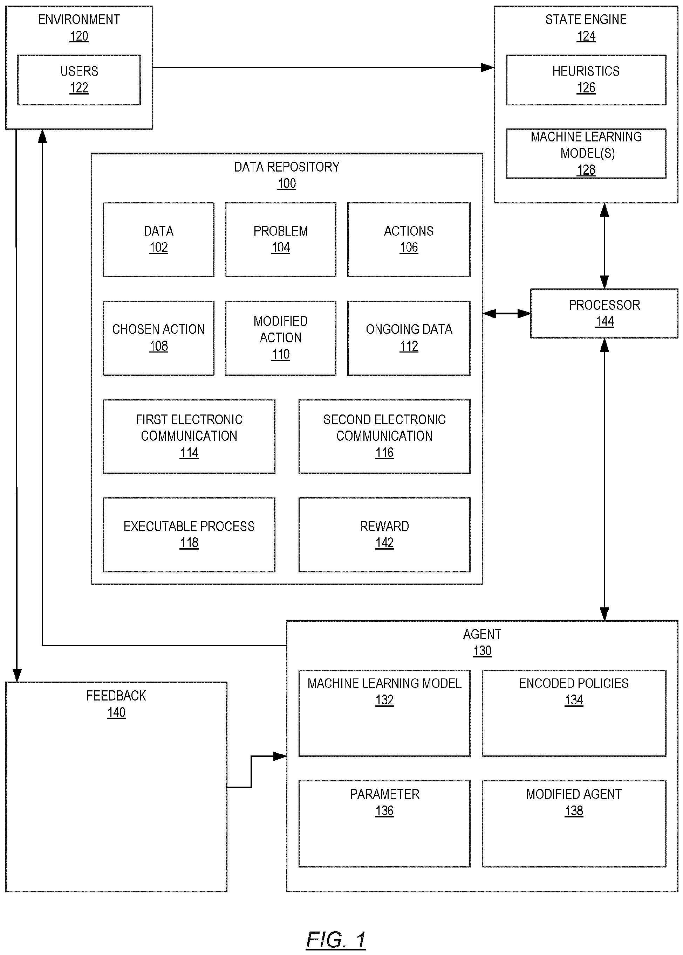

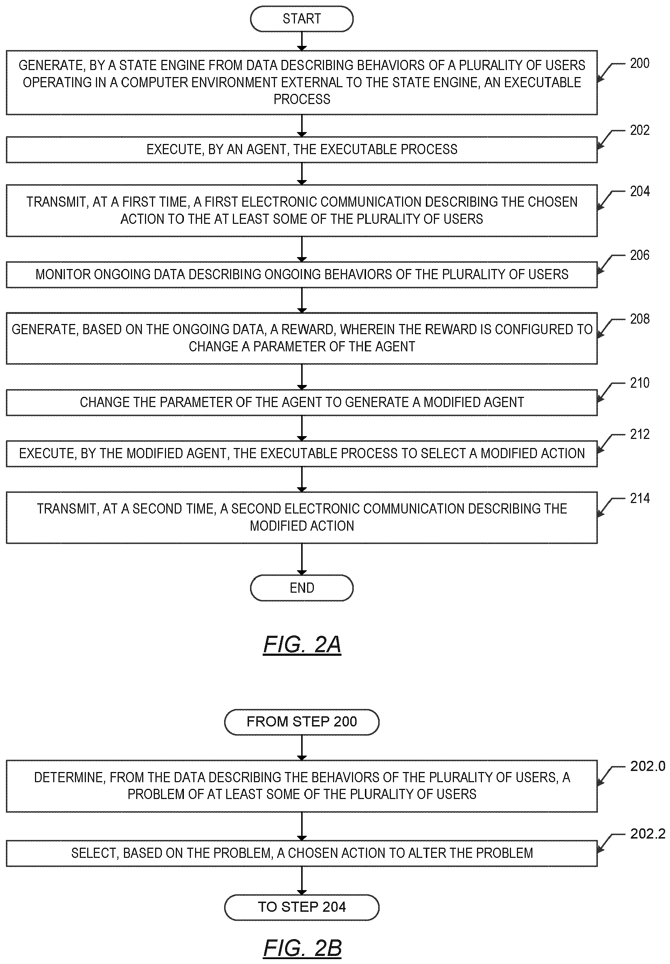

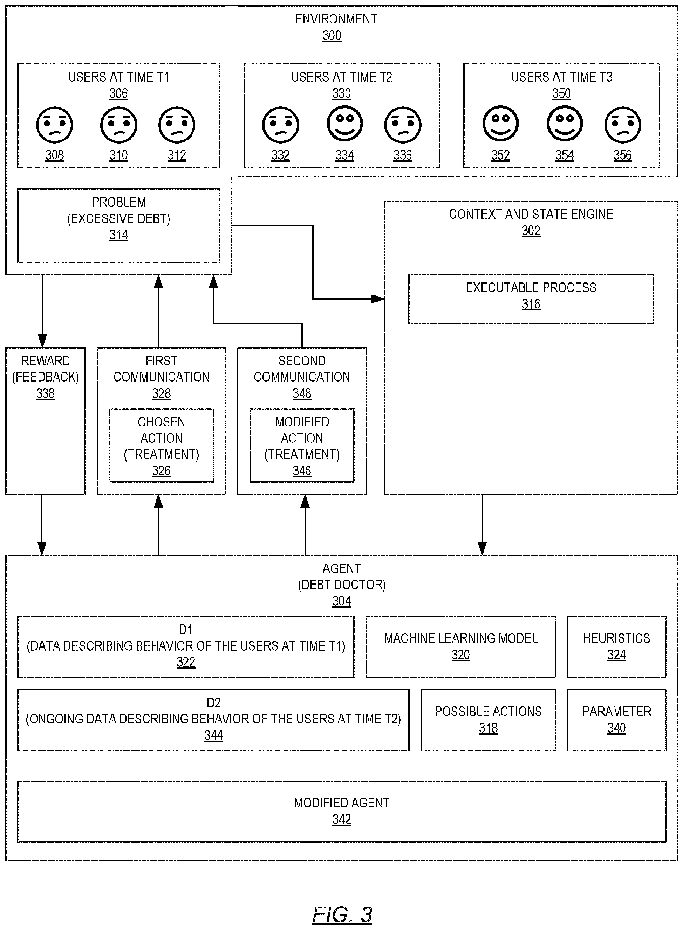

In other words, the one or more embodiments are directed to improving the functioning of computers by automatically improving the output of software systems. The one or more embodiments address the technical issue of automatically improving software by use of the feedback system, agent, and state engine described with respect to FIG. 1, FIG. 2A, and FIG. 2B. FIG. 3 provides an example of the one or more embodiments in use.

Attention is now turned to the figures. FIG. 1 shows a computing system for automatically updating software, in accordance with one or more embodiments. The computing system includes a data repository (100). In one or more embodiments, the data repository (100) is a storage unit and/or device (e.g., a file system, database, collection of tables, or any other storage mechanism) for storing data. Further, the data repository (100) may include multiple different storage units and/or devices. The multiple different storage units and/or devices may or may not be of the same type and may or may not be located at the same physical site. The data repository (100) may be a non-transitory computer readable storage medium.

The data repository (100) stores data (102). The data (102) describes behaviors of users (122) in an environment (120). The users (122) and the environment (120) are described further below. Examples of data include identifiers for the users (122), demographic information describing the users (122), usage data relating to a software application used by the users (122), and domain data pertaining to the users (122). Domain data is contextual data that is associated with the users (122) and is also associated with one or more behaviors of the users (122) that an operator of the system of FIG. 1 seeks to modify.

For example, the system of FIG. 1 may relate to improving the functioning of an agent (130) (described below) to modify the behavior of the users (122) with respect to financial decisions. Thus, for example, the domain data (and hence the data (102)) may include account balances of the users, debt trends, payment trends, behavioral biases, historical interactions between treatments and attributes of the users (122), interest fees paid by the users (122), insufficient funds fees paid by the users (122), late fees paid by the users (122), credit card debt of the users (122), and many other types of financial information.

In another example, the system of FIG. 1 may relate to improving the functioning of the agent (130) to modify the behavior of the users (122) with respect to academic studies. Thus, for example, the domain data (and hence the data (102)) may include recorded study hours, test scores, educational assessments, projects completed, grades received, and the like.

In still another example, the system of FIG. 1 may relate to improving the functioning of the agent (130) to modify the behavior of the users (122) with respect to medical conditions of the users (122). For example, the domain data (and hence the data (102)) may include the results of medical tests, patient vital statistics, health trends over time, treatments completed, and the like.

The data repository (100) also stores a problem (104) among possibly multiple problems. The problem (104) is a quantified attribute or pre-defined behavior that pertains to the users (122), where the quantified attribute is to be modified. The problem (104) attribute or pre-defined behavior may be deemed undesirable. For example, the problem (104) may be a degree of credit card debt, a credit score below a threshold value, a tendency to avoid paying minimum balances on time, *etc.* The problem (104) may be a health-related problem, such as a weight of the users (122) pre-determined to be obese, a blood sugar level deemed to be in a diabetic range, a tendency to eat sugary foods, *etc.* The problem (104) may be an academic-related problem, such as low test scores, a tendency to devote less than a threshold period of time to studies, *etc.*

The problem (104) may also be deemed desirable. For example, the problem (104) may be a high academic performance or more than the threshold period of time devoted to studies, which attributes or behaviors are to be retained. The problem (104) may be an assessment of an independent variable of a scientific experiment, in which case the users (122) could be deemed dependent variables that are tied to the independent variable, as explained further below.

Thus, the problem (104) is, most broadly, an quantified attribute that pertains to the users (122), where the quantified attribute is to be modified. In other words, the problem (104) is a property related to the users (122) that the agent (130) is designed to influence.

In an embodiment, the problem (104) is unknown, but quantifiable. For example, in a financial management context, the problem (104) may be a determination whether a user affected by a bias or exhibits negative behavior. The bias may be a self-control and present bias *(i.e.,* the user tends to purchase unneeded items impulsively) or an ostrich bias *(i.e.,* the user tends to ignore financial issues and thus forgets to pay minimum balances on time), and the like. The negative behavior may be spending beyond the user's income or unnecessarily paying more to receive a particular good or service. Many other examples are possible.

When the problem (104) is unknown, the problem (104) may still be quantified. For example, the agent (130) (described below) can predict or determine that a particular user in the users (122) is subject to an ostrich bias. Metadata associated with the user (such as an integer value) may be changed to indicate that that user is subject to an ostrich bias. In any case, the problem (104) is a quantified and measurable.

The data repository (100) also stores multiple actions (106). An action is a behavior, deed, or habit which one or more of the users (122) may perform or adopt. The actions (106) are pre-determined and depend on a context and purpose of the agent (130) (defined below). For example, in the financial management context, the actions (106) may include automatically saving money each month as a deduction from a paycheck, encouraging a user to check an account balance or to setup a payment reminder, a suggestion to reduce spending on a particular type of expense (e.g., eating at restaurants), and many other possible examples.

The data repository (100) also stores a chosen action (108). The chosen action (108) is one of the actions (106). However, the chosen action (108) is selected by the agent (130) for presentation to one or more of the users (122), as described with respect to FIG. 2A and FIG. 2B.

The data repository (100) also stores a modified action (110). The modified action (110) is one of the actions (106). However, the modified action (110) is selected by the agent (130) after the agent (130) has been modified as a result of feedback (140), as described with respect to FIG. 2A and FIG. 2B. In other words, the modified action (110) is one the actions (106) selected after the agent (130) has been improved.

The modified action (110) may take a variety of forms. In an embodiment, the modified action (110) is a modification to one of the actions (106). In an embodiment, the modified action (110) is a new action selected from among the actions (106), possibly unrelated to the chosen action (108). In an embodiment, the modified action (110) may be the same as the chosen action (108), but sent at a different time. For example, if a user is determined to have an ostrich bias (*i*.*e.* tends to hide from or ignore due dates), then the modified action (110) may be to transmit the chosen action (108) (*e.g.* a reminder) at a time selected to be more likely to prompt the user into action.

The data repository (100) also stores ongoing data (112). The ongoing data (112) is similar to the data (102) and may include the same data types as the data (102). However, the ongoing data (112) is obtained over time after the agent (130) has at least transmitted the first electronic communication (114) (defined below). The ongoing data (112) could include more or less data, or different data types, than the data (102). For example, if one or more of the actions (106) are of a pre-defined type of action, then some other form of data or additional data may be of interest when observing the future behavior of the users (122).

The data repository (100) also stores a first electronic communication (114). The first electronic communication (114) is an email, a pop-up window, a text, a social media posting, a dialog box, or some other computer-based mechanism for communicating with one or more of the users (122) via a computing device operated by the one or more of the users (122). As a specific example, the first electronic communication (114) could be a pop-up window or message displayed in a financial management software program used by the one or more of the users (122). However, the one or more embodiments contemplate many other forms of electronic communications.

In particular, the first electronic communication (114) is an initial electronic communication which contains the chosen action (108). Thus, the first electronic communication (114) is used to display the chosen action (108) to the one or more of the users (122). The one or more of the users (122) are free to adopt, reject, or modify the chosen action (108).

In addition, the data repository (100) also stores a second electronic communication (116). The second electronic communication (116) is like the first electronic communication (114); however, the second electronic communication (116) is sent after a pre-determined period of time. The pre-determined period of time is based on a time used to obtain feedback (140) (defined below) from the one or more of the users (122) and update the agent (130) (defined below). Specifically, the second electronic communication (116) is sent by the modified agent (138) (defined below).

Thus, the second electronic communication (116) contains the modified action (110). Like the first electronic communication (114), the second electronic communication (116) is used to display the modified action (110) to the one or more of the users (122). The users (122) are free to adopt, reject, or modify the modified action (110).

The data repository (100) also stores an executable process (118). The executable process (118) is a function, software algorithm, and/or a set of policies and/or other automated rules expressed as computer readable program code. The executable process (118) is programmed to identify a type of the problem (104) that applies to the one or more of the users (122), and then to select one or more of the actions (106) according to the type of the problem (104). For example, the executable process (118) may be programmed such that if a pattern of behavior of the one more of the users (122) indicates consistently making late payments and paying late fees, then those users are labeled for purposes of computing as having an ostrich bias. In turn, the executable process (118) then selects the chosen action (108) from the actions (106) that is pre-determined to assist the one or more of the users (122) overcome the ostrich bias. Additional details regarding the executable process (118) are described with respect to FIG. 2A and FIG. 2B.

The data repository (100) may also store a reward (142). The reward (142) is an instruction or a value that is used to modify the agent (130). For example, the reward (142) may be a weight that changes the output of the machine learning model (132) and/or the encoded policies (134) when the executable process (118) is executed. In another example, the reward (142) is an instruction or value that is used to train the machine learning model (132). Thus, for example, the reward (142) may be characterized as a loss function in some embodiments. A loss function is used to modify the machine learning model, which is then re-executed. A new result is compared to the known result. The process of training iterates until convergence. Convergence occurs when the output of the machine learning model being trained is within a threshold percentage of the known result, or when a certain number of iterations have occurred.

The reward (142) may also be a "reward" that is maximized using the reinforcement learning contextual bandits approach, described further below. The reward (142) is described further below with respect to the feedback (140), as well as with respect to the method of FIG. 2A and FIG. 2B.

The reward (142) may be based on a variety of different properties of the users (122). The term "based on" means that the reward (142) is derived in some manner from the property or properties. For example, different values of properties may change how the reward (142) is characterized, which in turn changes ow the reward (142) modifies the agent (130).

The reward (142) may be based on many different properties. The reward (142) may be based on click-through-rates of the users (122) on the chosen action (108). In other words, a measurement is taken regarding how quickly, how often, and whether the users (122) click on a widget presented in the first electronic communication (114) and/or the second electronic communication (116).

The reward (142) may also be based on a first measurement of a degree to which the users (122) adopted the chosen action (108). For example, if the users (122) adopt some or all of the chosen action (108), then the reward (142) is deemed to a greater or lesser extent to reinforce the output of the agent (130) to produce similar actions from the actions (106), or to advance the users (122) to a higher level of possible actions. In a specific example, if the users (122) adopt the chosen action (108) to pay minimum balances on time, then the chosen action (108) may be deemed successful. In this case, the reward (142) is calibrated to cause the agent (130) to select another of the actions (106) that prompts a modified action (110) from the users to pay more than the minimum balance.

The reward (142) may also be based on a second measurement of a degree to which the users (122) continue to have the problem (104) after the users (122) have viewed the chosen action (110). In a manner similar to that described immediately above, the reward (142) may be calibrated to change the agent (130) such that the modified agent (138) selects the modified action (110). In this case, the modified action (110) is an alternative action intended to attempt to modify the behavior of the users (122) in some other manner than that first attempted by the chosen action (108).

The reward (142) may also be a third measurement that the data (102) changes less than a first threshold amount after the users (122) have viewed the chosen action (108). The third measurement indicates that a first number of the users (122) continue to engage in a behavior pre-determined to be negative. The reward (142) is then used by the agent (130) as a basis to form the modified agent (138) to select the modified action (110).

The reward (142) may also be a fourth measurement that the data (102) changes more than a second threshold amount after the users (122) have viewed the chosen action (108). The fourth measurement indicates that a second number of the users (122) have adopted a new behavior related to the chosen action, the new behavior pre-determined to be positive. Again, the reward (142) is used by the agent (130) as a basis to form the modified agent (138) to select the modified action (110).

The reward (142) may also be structured on the basis of results, rather than user actions. For example, assume the users (122) followed the chosen action (108) as originally recommended, but then one or more of the users (122) later experienced a decrease in their measured financial status. In this case, the reward may be negative for those one or more of the users (122) that experienced the decrease in their measured financial status. As a result, that particular chosen action (108) will be disfavored for those one or more of the users (122) when the agent (130) selects new or modified actions (106).

Other types of the reward (142) are contemplated. For the reinforcement learning contextual bandits approach to the agent (130), the reward (142) may be positive rewards or negative rewards. Positive rewards reflect the users (122) adopting desired behaviors or achieving desired goals. Negative rewards reflect the users (122) failing to adopt desired behaviors or failing to achieve desired goals. Positive rewards reinforce the agent (130) to select for the modified action (110) new, modified, or repeated actions (106) that are similar to the chosen action (108). Negative rewards reinforce the agent (130) to select for the modified action (110) new or modified actions (106) that are different than the chosen action (108).

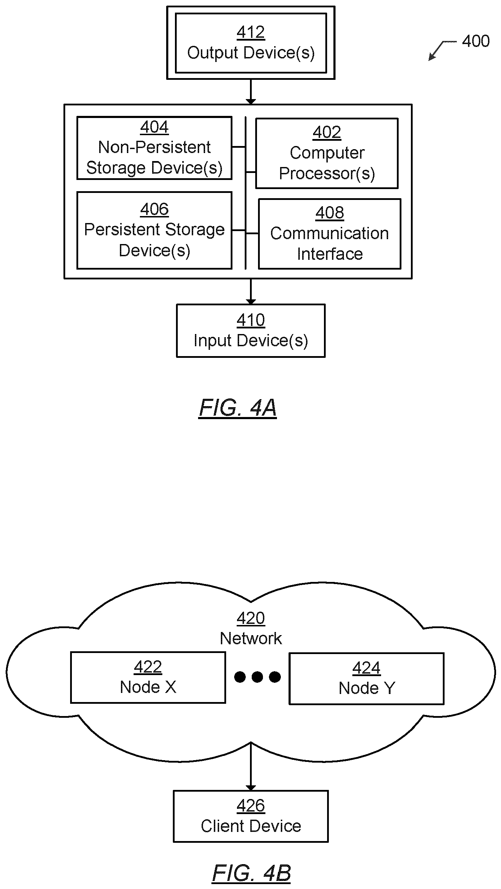

The system of FIG. 1 may also include an environment (120). The environment (120) is one or more user computing devices in a possibly distributed computing environment, such as described with respect to FIG. 4A and FIG. 4B. The environment (120) is used by the users (122).

The users (122) are the subjects of study or interest. The users (122) exhibit behaviors or properties that may be adjusted or changed over time. The system of FIG. 1 does not have direct control over the users (122). In other words, the users (122) are free to behave independently of the system of FIG. 1.

In one embodiment the users (122) are humans that use the computers in the environment (120) to accomplish some purpose. For example, the users (122) may be humans that use financial management software to manage their finances. In this case, the data (102) may be financially properties or behaviors of the users (122). The users (122) may be humans that use academic study skill software. In this case, the data (102) may be related to academic properties or behaviors of the users (122). The users (122) may be humans that have medical conditions. In this case the data (102) may be medical properties or medically-related behaviors of the users (122).

In another embodiment, the users (122) are not human. For example, the users (122) may be dependent variables in a scientific experiment. In this case, independent variables may be manipulated and changes to the users (122) *(i.e.* the dependent variables) observed for purposes of scientific inquiry. In this example, the data (102) relates to the dependent variables, and the agent (130) is used to draw conclusions regarding the reasons for changes in the dependent variables, or perhaps conclusions as to the nature of the independent variables.

The system of FIG. 1 includes other components. For example, the system of FIG. 1 also includes a state engine (124). The state engine (124) is configured to generate the executable process (118). In particular, the state engine (124) includes heuristics (126) and/or machine learning model(s) (128). In either or both cases, the state engine (124) is configured to take, as input, the data (102) and/or the ongoing data (112), and produce, as output, the executable process (118).

The heuristics (126) may be a set of rules and/or policies. The heuristics (126) may be characterized as software heuristics. The heuristics (126), when executed, generate, from the data (102) and/or the ongoing data (112), a set of automated rules relating the behaviors of the users (122) to outcomes of the behaviors for the users (122).

The machine learning model(s) (128) are supervised or unsupervised machine learning models. The machine learning model(s) (128), when executed, generate from the data (102) and/or the ongoing data (112), predictions or classifications of the users (122) with respect to categories into which the users may fall. Thus, for example, one machine learning model may predict whether the users (122) have the ostrich bias, another may predict whether the users (122) have a procrastination bias, *etc.* In this manner, the machine learning model may categorize the users into a variety of categorizations.

In an embodiment, the executable process (118) is stable over time, relative to the output of the agent (130). In other words, once the various types of the problem (104) or problems are known, and which of the actions (106) correspond to the problem (104) or problems, the executable process (118) does not change over time unless a reason exists to change the executable process (118).

However, the executable process (118) may be changed by the state engine (124) continuing to generate new versions of the executable process (118) based on the ongoing data (112). However, unless a threshold degree of change exists, which could be any change in one embodiment, the executable process (118) is not updated with respect to the use of the executable process (118) by the agent (130). Nevertheless, it is possible to update the executable process (118) as the overall statistical behavior of the users (122) changes over time.

The state engine (124) is external to the environment (120). In other words, the state engine (124) is a server-side application with which the users (122) do not interact directly.

The system shown in FIG. 1 also includes an agent (130). The agent (130) is a machine learning model (132) and/or one or more encoded policies (134). The agent (130) takes, as input, the data (102), executes the executable process (118), and produces, as output, the chosen action (108) and/or the modified action (110).

The machine learning model (132) may be one or more machine learning models. The machine learning model (132) may be of a variety of types, including an unsupervised machine learning model or a neural network. In an embodiment, the machine learning model (132) may be a contextual bandits reinforced learning machine learning model. In this case, the goal of the agent (130) is to mathematically maximize the rewards (142) that were given by the chosen action (108) in order to converge on the "best" policy. The "best" policy is one of the actions (106) that is most likely to produce a desired change in the behaviors or properties of the users (122).

Reinforcement learning is a machine learning paradigm used to train models for sequential decision making. Reinforcement learning involves using algorithms concerned with how a software agent takes suitable actions in complex environments and uses the feedback to maximize reward over time. Reinforcement learning provides the freedom to observe specific user behaviors, in a given context, and provide feedback on how the chosen behaviors are rewarded based on the goal. The contextual bandits approach relates to a flexible subset of reinforcement learning. The contextual bandit approach to reinforcement learning frames decision-making between separate actions in a given context.

The contextual bandits technique may be approached as a repeated game between two players, with every stage including three steps. First, the users (122) chooses k rewards r₁, ..., rₖ ∈ [0, 1]. Second, the agent (130) chooses an arm i E {1, k} without knowledge of users (122)'s chosen rewards. Third, the agent (130) observes the reward rᵢ. Further information regarding the operation of the agent (130) using the contextual bandits approach is described with respect to FIG. 2A and FIG. 2B.

The reinforcement learning contextual bandits approach is a particular embodiment of the machine learning model (132). However, the agent (130) may also be implemented as one or more encoded policies (134). The agent (130) may be operable to execute one or more aspects of the executable process (118), to determine the chosen action (108) and/or the modified action (110) based only on rules, *etc.* Thus, in some embodiments the agent (130) is the machine learning model (132), in some embodiments the agent (130) is the encoded policies (134), and in some embodiments the agent (130) is a combination of the machine learning model (132) and the encoded policies (134).

The agent (130) also includes a parameter (136). The term "parameter" may refer to multiple parameters of possibly different types. The parameter (136) is a setting of one or more of the machine learning model (132) and the encoded policies (134). For example, the parameter (136) could be a weight applied to the machine learning model (132) and/or the encoded policies (134). In another example, the parameter (136) may be the lambda parameter used in the contextual bandits reinforcement learning approach. Many other possible implements of the parameter (136) exist.

The function of the parameter (136) is to provide a means to alter the agent (130). When the parameter (136) changes, the agent (130) changes. Thus, when the parameter (136) changes, the output of the agent (130) changes.

Thus, by altering the parameter (136), the agent (130) may be transformed into the modified agent (138). The modified agent (138) is the agent (130), but with a parameter (136) that has been changed. Changing or selecting the parameter (136) is performed using feedback (140) as described with respect to FIG. 2A and FIG. 2B. In the system of FIG. 1, the modified agent (138) takes as input the ongoing data (112) and generates as output the modified action (110).

The system shown in FIG. 1 also includes feedback (140). The feedback (140) is an instruction to change the parameter (136) of the agent (130). The feedback (140) is generated based on the ongoing data (112) observed from the ongoing behavior of the users (122). The feedback (140) may be generated according to a number of different techniques, as described with respect to FIG. 2A and FIG. 2B. In the reinforcement learning contextual bandits example, the feedback (140) is the reward (142). The reward (142) is maximized over time via a convergence process, and the reward (142) is then used to select an adjustment to the parameter (136). Additional discussion of the generation and use of the feedback (140) is described with respect to FIG. 2A and FIG. 2B.

The system shown in FIG. 1 also includes a processor (144). The processor (144) is one or more computing devices in a possibly distributed computing environment, as described with respect to FIG. 4A and FIG. 4B. The processor (144) is in communication with the data repository (100), the state engine (124), the agent (130), and the feedback (140). The processor (144) may also have one or more communication devices for receiving or otherwise obtaining the data (102) and/or the ongoing data (112) from the environment (120), for storage in the data repository (100).

While FIG. 1 shows a configuration of components, other configurations may be used without departing from the scope. For example, various components may be combined to create a single component. As another example, the functionality performed by a single component may be performed by two or more components.

FIG. 2A and FIG. 2B are flowcharts of a method of automatically updating software, in accordance with one or more embodiments. The methods of FIG. 2A and FIG. 2B may be implemented using the system of FIG. 2A and FIG. 2B, possibly using one or more of the components in the computing system and network environment of FIG. 4A and FIG. 4B. The methods of FIG. 2A and FIG. 2B may be characterized as a method of auto-improving a software system for user behavior modification. The methods of FIG. 2A and FIG. 2B may be combined and executed as a single method, as explained with respect to FIG. 2B.

Attention is first turned to FIG. 2A. Step 200 includes generating, by a state engine from data describing behaviors of users operating in a computer environment external to the state engine, an executable process. The executable process is generated according to a number of machine learning models and/or heuristics as defined with respect to FIG. 1. The state engine takes, as input, the data and produces as output the executable process.

Step 202 includes executing, by an agent, the executable process. The executable process is executed by an agent. The agent takes as input the data and/or ongoing data regarding the users. The agent, using the executable process, produces as output a chosen action from among the available pre-determined actions.

Step 204 includes transmitting, at a first time, a first electronic communication describing the chosen action to the at least some of the users. The first electronic communication is transmitted to one or more of the users in the user environment. For example, the agent may be a software module connected to a financial management software being used by the users in the environment. In this case, the agent may present a pop-up window to the selected users in the environment while they use the financial management software. In another example, the agent may transmit an email or a text message to the selected users.

Step 206 includes monitoring ongoing data describing ongoing behaviors of the users. Monitoring the ongoing data may be performed by the agent or by some other data collection process. In either case, the ongoing data is retained in a data repository, or may be added to the existing data.

Step 208 includes generating, based on the ongoing data, a reward, wherein the reward is configured to change a parameter of the agent. The reward is generated by using the data to create a penalty or a reinforcement that is applied to the agent. For example, if the users ignore the chosen action in the first electronic communication, then the reward may be a weight selected to cause the agent to be less likely to select the first chosen action. In another example, if the users respond well to the chosen action in the first electronic communication, then the reward may be a weight selected to cause the agent to continue to select the first chosen action. Alternatively, the reward may be a flag that causes the agent to be able to select a more advanced selected action for presentation to uses who are responsive to the first chosen action.

As mentioned above, in an embodiment the agent may use a reinforcement learning contextual bandits machine learning approach. In this case, the reward is maximized by the agent itself. Initially, the machine learning algorithm carries out the three steps mentioned in FIG. 1 for the contextual bandits reinforced learning approach.

The agent then interacts with the context (e.g. the data or the ongoing data) of a user's behavior (search history, visited pages, or geolocation) in the environment. The agent then performs the following functions. Some context, "x" (*e.g.* ongoing data) is observed by the agent. The agent then chooses another action, "a," from a set of actions "A" *(i.e.,* a E A (A may depend on x)). Some reward, "r," for the chosen "a" is observed by agent.

The agent selects actions that provide the highest possible reward. In other words, what is desired is a modified agent that selects an optimized action to take. Contexts and actions are typically represented as feature vectors in contextual bandit algorithms. For example, the agent chooses actions by applying a policy, "π," that takes a context as input and returns an action. The agent is programmed to find a policy that maximizes the average reward, "r," over a sequence of interactions.

As shown above, multiple techniques exist for generating the reward at step 208. Other techniques are possible, such as by using some other heuristics or machine learning algorithms external to the agent. Thus, the reward generation and action selection process may be a combination of multiply interacting machine learning models.

Step 210 includes changing the parameter of the agent to generate a modified agent. The penalty or the reward modifies the parameter of agent. For example, if the parameter is a weight, then the weight is applied to the logic *(i.e.,* the executable process and/or other logic) executed by the agent. In the context of the reinforced learning contextual bandits approach, the reward is inserted into the reinforced learning algorithm.

However the parameter is changed by the reward, the result is a modified agent. The agent is modified and is different than the prior iteration of the agent. Thus, the agent is automatically improved over time and becomes better at influencing user behavior. The agent is improved in that the agent is better able to select actions more likely to prompt a desired response from the users.

Step 212 includes executing, by the modified agent, the executable process to select a modified action. The modified agent executes the executable process again, this time with the modified parameter or parameters. As a result, the modified agent selects a modified action in a manner consistent with the previously determined reward.

Step 214 includes transmitting, at a second time, a second electronic communication describing the modified action. The second electronic action may be transmitted in a manner similar to the transmission of the first electronic communication at step 204.

However, the second electronic communication may be transmitted in a manner different than the first electronic communication. For example, the modified action may be to transmit the chosen action at a different time of day or a different day of the month. Thus, the timing of when the second time is selected is calculated to be more likely to influence the one or more users. The modified action may be to transmit the chosen action in a different manner. For example, if the first second electronic communication was transmitted via a text, then the second electronic communication may be transmitted via a pop-up window. In still another example, the second electronic communication may transmit an entirely different proposed action, in which case the relative timing and/or method of transmission of the second electronic communication may be varied.

Still other variations are possible. In any case, the second time may be selected by the agent to increase a probability that the at least some of the users adopt a new behavior suggested by the modified action in the second electronic communication.

Attention is now turned to FIG. 2B. The method of FIG. 2B represents steps taken as part of executing the executable process at step 202 of FIG. 2A. Thus, the method of FIG. 2A and the method of FIG. 2B may be combined and executed as a single method.

Step 202.0 includes determining, from the data describing the behaviors of the users, a problem of at least some of the users. The determination of the problem is automatically performed by the heuristics and/or machine learning models of the state engine. The problem is determined by selecting from among a number of pre-determined problems. Such problems may include self-control problems, present bias problems, ostrich bias problems, procrastination problems, limited attention problems, literacy problems, fee payment problems, and possibly many others.

The exact nature of the problem and the list of problems to select from depend on the implementation of the system. For example, if the system is directed to influencing health-related behaviors of the users, then a different set of problems may be defined and tracked from those described above.

Step 202.2 includes selecting, based on the problem, a chosen action to alter the problem. The chosen action is selected from among a number of pre-defined actions. For example, if the problem is selected to be obesity in a user, then, further based on demographic characteristics of the user in question, the chosen action may be to suggest that the user substitute one meal a day with a plain, raw salad. In another example, if the problem is selected to be excessive debt and a sub-problem of the user in question is ostrich bias, then the selected action may be to remind the user to make a payment at a pre-determined time when the user is believed to be more available or receptive.

The chosen action may be more sophisticated. For example, if the user does not have financial liquidity challenges, but nevertheless has excessive debt, then the chosen action may be to suggest that the user restructures the user's debts. The suggestion to restructure may be accompanied by a link to a debt consolidation loan program offered by the company that manages the financial management software of the environment as well as the automatically improving software system described herein. The chosen action may also be layered or contingent. For example, once the user accomplishes one action, a subsequent action is automatically suggested to the user.

The chosen action need not be directly related to the problem. For example, a user having excessive debt may be directed to a stress management program in order to curb excessive buying habits that developed as a result of excessive stress in the user's life.

While the various steps in the flowcharts of FIG. 2A and FIG. 2B are presented and described sequentially, one of ordinary skill will appreciate that some or all of the steps may be executed in different orders, may be combined or omitted, and some or all of the steps may be executed in parallel. Furthermore, the steps may be performed actively or passively. For example, some steps may be performed using polling or be interrupt driven in accordance with one or more embodiments. By way of an example, determination steps may not require a processor to process an instruction unless an interrupt is received to signify that condition exists in accordance with one or more embodiments. As another example, determination steps may be performed by performing a test, such as checking a data value to test whether the value is consistent with the tested condition in accordance with one or more embodiments. Thus, the one or more embodiments are not necessarily limited by the examples provided herein.

FIG. 3 presents a specific example of the techniques described above with respect to FIG. 1, FIG. 2A, and FIG. 2B. The following example is for explanatory purposes only and not intended to limit the scope.

In the example of FIG. 3, the environment (300) is a financial management application (FMA) that is accessed via network connections by a variety of individual personal computers operated by users. The context and state engine (302) and the agent (304) are part of the environment (300), though the various components of the FMA are not shown in order to maintain clarity in the example. One or more processors (such as the processor (144) in FIG. 1) execute the FMA, the context and state engine (302), and the agent (304), but again is not shown for clarity of the operation of the example.

In the example of FIG. 3, the users at time T1 (306) are users of the FMA. The users at time T1 (306) include User A_{T1} (308), User B_{T1} (310), and User Cn (312). All three users have a problem (314): excessive debt. The problem (314) is a quantified assessment. Here, excessive debt means that the users at time T1 (306) each have debt-to-income ratios above a pre-determined threshold value.

An automatically updating software program, the agent (304), is used to suggest alterations to the behaviors of the User An (308), the User B_{T1} (310), and the User Cn (312). The agent (304) may be characterized in the example as a "debt doctor," though the agent (304) is software and not a human. Each separate user may receive the same or different chosen actions from the agent (304), depending on the individual context of the individual user.

In the example, prior to time T1, the context and state engine (302) has generated an executable process (316). The executable process (316) is an executable file and/or a set of relations in vector format. The executable process (316) is suitable for input to or execution by the agent (304). The executable process (316) is configured to allow the agent (304) to predict and/or select from among a number of possible actions (318). Each of the possible actions (318) addresses a distinct problem.

Note that while the users at time T1 (306) all have the same problem (314) of excessive debt, not all of the users at time T1 (306) have the same sub-problems. For example, the User A_{T1} (308) has a problem with ostrich bias (*i.e.* the User A_{T1} (308) hopes that the user's financial problems will go away without the user tending to the user's finances). The User B_{T1} (310) has a procrastination bias because the User B_{T1} (310) is busy and infrequently visits the FMA. The User Cn (312) has compulsive buying habit.

In each case, the executable process (316) may be executed to identify the relevant sub-problem for each user. In other words, the executable process (316) may be executed by the agent (304) to identify a "diagnosis" (sub-problem) for the "condition" (problem of excessive debt), and then to recommend a "treatment" (chosen action) that will address the diagnosis and thereby mitigate the condition.

In the example, the agent (304) includes a machine learning model (320). The machine learning model (320) is a reinforced learning model that uses the contextual bandits approach, as described with respect to FIG. 1 and FIG. 2.

The agent (304) executes the executable process (316) by inputting the vectors created by the context and state engine (302) to the machine learning model (320). The vectors include context related to the users at time T1 (306), and thus include at least some of D1 (322), which is defined as data describing the behavior of the users at time T1 (306). The machine learning model (320) classifies the users at time T1 (306) into different categories of sub-problem. Stated differently, the machine learning model (320) "diagnoses" the underlying cause of the problem (314) shared by the users at time T1 (306).

The agent (304) then executes heuristics (324) which relate the classification (*i.e.* diagnosis) to a chosen one of the possible actions (318). The heuristics (324) may also take into account some of the D1 (322) data that relates to the users at time T1 (306) when selecting the chosen action from the possible actions (318). Note that in some other example, the chosen action could be multiple actions, contingent actions, *etc.*, as described with respect to FIG. 2A and FIG. 2B.

The chosen action (326) (*i.e.*, the suggested "treatment"), individual to each of the users at time T1 (306), is transmitted to the users via a first communication (328). The first communication (328) is selected in terms of timing and form according to the heuristics (324) to maximize a probability that the corresponding user will respond positively to the chosen action (326). Thus, for example, the first communication (328) to the User An (308) may be a suggestion to address upcoming payments due. The first communication (328) to the User B_{T1} (310) may be to check in the FMA and manage the user's finances. The first communication (328) may be to reduce spending in a selected spending category as tracked in the FMA (*e.g.*, to reduce spending at restaurants).

As time passes, the users' online behavior in the environment (300) is monitored. At some pre-selected time later, T2, a status of the users is checked. Thus, the users at time T2 (330) include User A_{T2} (332), User B_{T2} (334), and User C_{T2} (336). Ongoing data regarding the users at time T2 (330) has been collected. The User A_{T2} (332) and the User C_{T2} (336) still have the problem (314); however, the User B_{T2} (334) no longer has the problem (314).

The agent (304) then generates a reward (338). The reward (338) is feedback configured to adjust a parameter (340) of the agent (304), and in particular the machine learning model (320). The reward (338) in this case adjusts the parameter, "r" used in the reinforced learning contextual bandits approached described with respect to FIG. 1, FIG. 2A, and FIG. 2B.

The application of the reward (338) changes the parameter (340), thereby producing a modified agent (342). The modified agent (342) is the agent (304), but now modified because the parameter (340) has been adjusted by the reward (338). Optionally, the machine learning model (320) may be re-trained using the adjusted parameter.

The agent (304) then re-executes the executable process (316), but this time taking D2 (344) as input. The D2 (344) is ongoing data describing the behavior of the users at time T2 (330).

As a result, the machine learning model (320) re-classifies the users at time T2 (330). Based on the D2 (344), and a lack of click-through data from the D1 (322), the User A_{T2} (332) is predicted to still have the ostrich bias, and the agent (304) is programmed to conclude that the User A_{T2} (332) ignored the first communication (328). Based on the D2 (344) and a log entry of the User B_{T2} (334) signing into the FMA and managing the finances of the User B_{T2} (334), the agent (304) is programmed to conclude that the first communication (328) was successful with the User B_{T2} (334). Based on the D2 (344) and increased buying at restaurants, the agent (304) is programmed to conclude that the User C_{T2} (336) is unable to reduce spending at restaurants for whatever reason (*e.g.*, the User C_{T2} (336) relies on taking business clients out to restaurants in order to generate revenue).

The agent (304) then executes the heuristics (324) to select a modified action (346) (*i.e.*, a newly determined "treatment"). The modified action (346) is communicated individually to the users at time T2 (330) via a second communication (348). In the example, the modified action (346) for the User A_{T2} (332) is to change when the second communication (348) is delivered to the User A_{T2} (332) (*e.g.*, after work when the user is more likely to respond). The modified action (346) for the User B_{T2} (334) is to continue to send reminders at the same time, as the prior action is deemed to be successful at addressing the problem (314) for the User B_{T2} (334). The modified action (346) for the User C_{T2} (336) is to suggest a reduction in spending in a different area of the user's finances (e.g. in groceries, as the user's grocery spending is higher than average for the user's demographics).

Again, more time passes. The agent (304) continues to monitor additional data and generate additional feedback to continue to modify the agent (304). Thus, for example, the agent (304) may record the status of the users at time T3 (350). The agent (304) records that the User A_{T3} (352) no longer has the problem (314). The agent (304) records that the User B_{T3} (354) also no longer has the problem (314). The agent (304) records that the User C_{T3} (356) continues to have the problem (314), but not to the same degree as before (*i.e.*, the User C_{T3} (356) has made progress at reducing the user's debt to income ratio).

Thus, the process of feedback, modified actions, and new electronic communications continues. The iterative process is designed to improve the finances of the users of the FMA.

The example of FIG. 3 may thus be defined as a FMA software extension. In this case, the problem (314) is characterized as having a first pre-defined financial behavior. The chosen action (326) is a second pre-defined financial behavior, different than the first pre-defined financial behavior. The ongoing data (D2 (344)) includes changes to the user's measured financial data. The modified action (346) is configured improve a probability that the users will at least adopt the second pre-defined financial behavior.

The example of FIG. 3 may be varied. The one or more embodiments are not directed necessarily towards a financial management problem. The automatically improving software system for behavior modification of the one or more embodiments may also be used in other applications.

For example, the problem (314) may be a first pre-defined academic behavior. In this case, the chosen action (326) is a second pre-defined academic behavior, different than the first pre-defined academic behavior. The ongoing data (D2 (344)) includes changes to measured academic data describing measured academic performance of the users. The modified action (346) is configured improve a probability that the users will at least adopt the second pre-defined academic behavior.

In yet another example, the problem (314) is a first pre-defined health-related behavior. In this case, the chosen action (326) is a second pre-defined health-related behavior, different than the first pre-defined health-related behavior. The ongoing data (D2 (344)) includes changes to measured medical data describing measured medical states of users. The modified action (346) is configured improve a probability that the users will at least adopt the second pre-defined health-related behavior.

Thus, the one or more embodiments generally improve a computer as a tool by programming the computer to automatically improve the software executing on the computer. Other alternative applications are possible, and so the examples described with respect to FIG. 3 do not necessarily limit the claims or other examples described herein.

FIG. 4A and FIG. 4B are examples of a computing system and a network, in accordance with one or more embodiments. Embodiments may be implemented on a computing system specifically designed to achieve an improved technological result. When implemented in a computing system, the features and elements of the disclosure provide a significant technological advancement over computing systems that do not implement the features and elements of the disclosure. Any combination of mobile, desktop, server, router, switch, embedded device, or other types of hardware may be improved by including the features and elements described in the disclosure. For example, as shown in FIG. 4A, the computing system (400) may include one or more computer processor(s) (402), non-persistent storage device(s) (404) (e.g., volatile memory, such as random access memory (RAM), cache memory), persistent storage device(s) (406) (e.g., a hard disk, an optical drive such as a compact disk (CD) drive or digital versatile disk (DVD) drive, a flash memory, etc.), a communication interface (408) (e.g., Bluetooth interface, infrared interface, network interface, optical interface, etc.), and numerous other elements and functionalities that implement the features and elements of the disclosure.

The computer processor(s) (402) may be an integrated circuit for processing instructions. For example, the computer processor(s) (402) may be one or more cores or micro-cores of a processor. The computing system (400) may also include one or more input device(s) (410), such as a touchscreen, a keyboard, a mouse, a microphone, a touchpad, an electronic pen, or any other type of input device.

The communication interface (408) may include an integrated circuit for connecting the computing system (400) to a network (not shown) (e.g., a local area network (LAN), a wide area network (WAN) such as the Internet, a mobile network, or any other type of network) and/or to another device, such as another computing device.

Further, the computing system (400) may include one or more output device(s) (412), such as a screen (e.g., a liquid crystal display (LCD), a plasma display, a touchscreen, a cathode ray tube (CRT) monitor, a projector, or other display device), a printer, an external storage, or any other output device. One or more of the output device(s) (412) may be the same or different from the input device(s) (410). The input and output device(s) (410 and 412) may be locally or remotely connected to the computer processor(s) (402), the non-persistent storage device(s) (404) , and the persistent storage device(s) (406). Many different types of computing systems exist, and the aforementioned input and output device(s) (410 and 412) may take other forms.

Software instructions in the form of computer readable program code to perform various embodiments may be provided by way of a computer-readable medium. A computer-readable medium may include a computer-readable storage medium and/or a computer-readable transmission medium. A computer readable storage medium (which may be termed "non-transitory") may store, in whole or in part, temporarily or permanently, on a physical medium such as a CD, a DVD, a storage device, a diskette, a tape, flash memory, physical memory, or any other computer readable storage medium. A computer-readable transmission may include a carrier wave, transmission signal or the like, and may occur between components of a single computer system and/or between plural separate computer systems. Specifically, the software instructions may correspond to computer readable program code that, when executed by a processor(s), is configured to perform one or more embodiments.

The computing system (400) in FIG. 4A may be connected to or be a part of a network. For example, as shown in FIG. 4B, the network (420) may include multiple nodes (e.g., node X (422), node Y (424)). Each node may correspond to a computing system, such as the computing system (400) shown in FIG. 4A, or a group of nodes combined may correspond to the computing system (400) shown in FIG. 4A. By way of an example, embodiments may be implemented on a node of a distributed system that is connected to other nodes. By way of another example, embodiments may be implemented on a distributed computing system having multiple nodes, where one or more portions of the disclosed techniques may be located on a different node within the distributed computing system. Further, one or more elements of the aforementioned computing system (400) may be located at a remote location and connected to the other elements over a network.

Although not shown in FIG. 4B, the node may correspond to a blade in a server chassis that is connected to other nodes via a backplane. By way of another example, the node may correspond to a server in a data center. By way of another example, the node may correspond to a computer processor or micro-core of a computer processor with shared memory and/or resources.

The nodes (e.g., node X (422), node Y (424)) in the network (420) may be configured to provide services for a client device (426). For example, the nodes may be part of a cloud computing system. The nodes may include functionality to receive requests from the client device (426) and transmit responses to the client device (426). The client device (426) may be a computing system, such as the computing system (400) shown in FIG. 4A. Further, the client device (426) may include and/or perform all or a portion of one or more embodiments of the described techniques.

The computing system (400) or group of computing systems described in FIG. 4A and 4B may include functionality to perform a variety of operations disclosed herein. For example, the computing system(s) may perform communication between processes on the same or different system. A variety of mechanisms, employing some form of active or passive communication, may facilitate the exchange of data between processes on the same device. Examples representative of these inter-process communications include, but are not limited to, the implementation of a file, a signal, a socket, a message queue, a pipeline, a semaphore, shared memory, message passing, and a memory-mapped file. Further details pertaining to a couple of these non-limiting examples are provided below.

Based on the client-server networking model, sockets may serve as interfaces or communication channel end-points enabling bidirectional data transfer between processes on the same device. Foremost, following the client-server networking model, a server process (e.g., a process that provides data) may create a first socket object. Next, the server process binds the first socket object, thereby associating the first socket object with a unique name and/or address. After creating and binding the first socket object, the server process then waits and listens for incoming connection requests from one or more client processes (e.g., processes that seek data). At this point, when a client process wishes to obtain data from a server process, the client process starts by creating a second socket object. The client process then proceeds to generate a connection request that includes at least the second socket object and the unique name and/or address associated with the first socket object. The client process then transmits the connection request to the server process. Depending on availability, the server process may accept the connection request, establishing a communication channel with the client process, or the server process, busy in handling other operations, may queue the connection request in a buffer until server process is ready. An established connection informs the client process that communications may commence. In response, the client process may generate a data request specifying the data that the client process wishes to obtain. The data request is subsequently transmitted to the server process. Upon receiving the data request, the server process analyzes the request and gathers the requested data. Finally, the server process then generates a reply including at least the requested data and transmits the reply to the client process. The data may be transferred, more commonly, as datagrams or a stream of characters (e.g., bytes).

Shared memory refers to the allocation of virtual memory space in order to substantiate a mechanism for which data may be communicated and/or accessed by multiple processes. In implementing shared memory, an initializing process first creates a shareable segment in persistent or non-persistent storage. Post creation, the initializing process then mounts the shareable segment, subsequently mapping the shareable segment into the address space associated with the initializing process. Following the mounting, the initializing process proceeds to identify and grant access permission to one or more authorized processes that may also write and read data to and from the shareable segment. Changes made to the data in the shareable segment by one process may immediately affect other processes, which are also linked to the shareable segment. Further, when one of the authorized processes accesses the shareable segment, the shareable segment maps to the address space of that authorized process. Often, only one authorized process may mount the shareable segment, other than the initializing process, at any given time.

Other techniques may be used to share data, such as the various data described in the present application, between processes without departing from the scope. The processes may be part of the same or different application and may execute on the same or different computing system.

Rather than or in addition to sharing data between processes, the computing system performing one or more embodiments may include functionality to receive data from a user. For example, in one or more embodiments, a user may submit data via a graphical user interface (GUI) on the user device. Data may be submitted via the graphical user interface by a user selecting one or more graphical user interface widgets or inserting text and other data into graphical user interface widgets using a touchpad, a keyboard, a mouse, or any other input device. In response to selecting a particular item, information regarding the particular item may be obtained from persistent or non-persistent storage by the computer processor. Upon selection of the item by the user, the contents of the obtained data regarding the particular item may be displayed on the user device in response to the user's selection.

By way of another example, a request to obtain data regarding the particular item may be sent to a server operatively connected to the user device through a network. For example, the user may select a uniform resource locator (URL) link within a web client of the user device, thereby initiating a Hypertext Transfer Protocol (HTTP) or other protocol request being sent to the network host associated with the URL. In response to the request, the server may extract the data regarding the particular selected item and send the data to the device that initiated the request. Once the user device has received the data regarding the particular item, the contents of the received data regarding the particular item may be displayed on the user device in response to the user's selection. Further to the above example, the data received from the server after selecting the URL link may provide a web page in Hyper Text Markup Language (HTML) that may be rendered by the web client and displayed on the user device.

Once data is obtained, such as by using techniques described above or from storage, the computing system, in performing one or more embodiments, may extract one or more data items from the obtained data. For example, the extraction may be performed as follows by the computing system (400) in FIG. 4A. First, the organizing pattern (e.g., grammar, schema, layout) of the data is determined, which may be based on one or more of the following: position (e.g., bit or column position, Nth token in a data stream, etc.), attribute (where the attribute is associated with one or more values), or a hierarchical/tree structure (consisting of layers of nodes at different levels of detail-such as in nested packet headers or nested document sections). Then, the raw, unprocessed stream of data symbols is parsed, in the context of the organizing pattern, into a stream (or layered structure) of tokens (where each token may have an associated token "type").

Next, extraction criteria are used to extract one or more data items from the token stream or structure, where the extraction criteria are processed according to the organizing pattern to extract one or more tokens (or nodes from a layered structure). For position-based data, the token(s) at the position(s) identified by the extraction criteria are extracted. For attribute/value-based data, the token(s) and/or node(s) associated with the attribute(s) satisfying the extraction criteria are extracted. For hierarchical/layered data, the token(s) associated with the node(s) matching the extraction criteria are extracted. The extraction criteria may be as simple as an identifier string or may be a query presented to a structured data repository (where the data repository may be organized according to a database schema or data format, such as eXtensible Markup Language (XML)).

The extracted data may be used for further processing by the computing system. For example, the computing system (400) of FIG. 4A, while performing one or more embodiments, may perform data comparison. Data comparison may be used to compare two or more data values (e.g., A, B). For example, one or more embodiments may determine whether A > B, A = B, A != B, A < B, etc. The comparison may be performed by submitting A, B, and an opcode specifying an operation related to the comparison into an arithmetic logic unit (ALU) (i.e., circuitry that performs arithmetic and/or bitwise logical operations on the two data values). The ALU outputs the numerical result of the operation and/or one or more status flags related to the numerical result. For example, the status flags may indicate whether the numerical result is a positive number, a negative number, zero, etc. By selecting the proper opcode and then reading the numerical results and/or status flags, the comparison may be executed. For example, in order to determine if A > B, B may be subtracted from A (i.e., A - B), and the status flags may be read to determine if the result is positive (i.e., if A > B, then A - B > 0). In one or more embodiments, B may be considered a threshold, and A is deemed to satisfy the threshold if A = B or if A > B, as determined using the ALU. In one or more embodiments, A and B may be vectors, and comparing A with B requires comparing the first element of vector A with the first element of vector B, the second element of vector A with the second element of vector B, etc. In one or more embodiments, if A and B are strings, the binary values of the strings may be compared.

The computing system (400) in FIG. 4A may implement and/or be connected to a data repository. For example, one type of data repository is a database. A database is a collection of information configured for ease of data retrieval, modification, re-organization, and deletion. Database Management System (DBMS) is a software application that provides an interface for users to define, create, query, update, or administer databases.

The user, or software application, may submit a statement or query into the DBMS. Then the DBMS interprets the statement. The statement may be a select statement to request information, update statement, create statement, delete statement, etc. Moreover, the statement may include parameters that specify data, data containers (a database, a table, a record, a column, a view, etc.), identifiers, conditions (comparison operators), functions (e.g. join, full join, count, average, etc.), sorts (e.g. ascending, descending), or others. The DBMS may execute the statement. For example, the DBMS may access a memory buffer, a reference or index a file for read, write, deletion, or any combination thereof, for responding to the statement. The DBMS may load the data from persistent or non-persistent storage and perform computations to respond to the query. The DBMS may return the result(s) to the user or software application.

The computing system (400) of FIG. 4A may include functionality to present raw and/or processed data, such as results of comparisons and other processing. For example, presenting data may be accomplished through various presenting methods. Specifically, data may be presented through a user interface provided by a computing device. The user interface may include a GUI that displays information on a display device, such as a computer monitor or a touchscreen on a handheld computer device. The GUI may include various GUI widgets that organize what data is shown as well as how data is presented to a user. Furthermore, the GUI may present data directly to the user, e.g., data presented as actual data values through text, or rendered by the computing device into a visual representation of the data, such as through visualizing a data model.

For example, a GUI may first obtain a notification from a software application requesting that a particular data object be presented within the GUI. Next, the GUI may determine a data object type associated with the particular data object, e.g., by obtaining data from a data attribute within the data object that identifies the data object type. Then, the GUI may determine any rules designated for displaying that data object type, e.g., rules specified by a software framework for a data object class or according to any local parameters defined by the GUI for presenting that data object type. Finally, the GUI may obtain data values from the particular data object and render a visual representation of the data values within a display device according to the designated rules for that data object type.

Data may also be presented through various audio methods. In particular, data may be rendered into an audio format and presented as sound through one or more speakers operably connected to a computing device.

Data may also be presented to a user through haptic methods. For example, haptic methods may include vibrations or other physical signals generated by the computing system. For example, data may be presented to a user using a vibration generated by a handheld computer device with a predefined duration and intensity of the vibration to communicate the data.

The above description of functions presents only a few examples of functions performed by the computing system (400) of FIG. 4A and the nodes (e.g., node X (422), node Y (424)) and/or client device (426) in FIG. 4B. Other functions may be performed using one or more embodiments of the disclosed techniques.

Therefore, from one perspective, there have been provided approaches that include generating, by a state engine from data describing behaviors of users in an environment external to the state engine, an executable process. An agent executes the executable process by determining, from the data describing the behaviors of the users, a problem of at least some of the users, and selects, based on the problem, a chosen action to alter the problem. At a first time, a first electronic communication describing the chosen action to the at least some of the users is transmitted. Ongoing data describing ongoing behaviors of the users is monitored. A reward is generated based on the ongoing data to change a parameter of the agent. The parameter of the agent is changed to generate a modified agent. The modified agent executes the executable process to select a modified action. At a second time, a second electronic communication describing the modified action is transmitted.

Further examples are set out in the following numbered clauses:
Clause 1. A method comprising: generating, by a state engine from data describing behaviors of a plurality of users operating in a computer environment external to the state engine, an executable process; executing, by an agent, the executable process, by: determining, from the data describing the behaviors of the plurality of users, a problem of at least some of the plurality of users, and selecting, based on the problem, a chosen action to alter the problem; transmitting, at a first time, a first electronic communication describing the chosen action to the at least some of the plurality of users; monitoring ongoing data describing ongoing behaviors of the plurality of users; generating, based on the ongoing data, a reward, wherein the reward is configured to change a parameter of the agent; changing the parameter of the agent to generate a modified agent; executing, by the modified agent, the executable process to select a modified action; transmitting, at a second time, a second electronic communication describing the modified action.
Clause 2. The method of clause 1, wherein the second time is selected by the agent to increase a probability that the at least some of the plurality of users adopt a new behavior suggested by the modified action in the second electronic communication.
Clause 3. The method of clause 1 or 2, wherein: the agent comprises a machine learning model, an input of the machine learning model comprises the data, and an output of the machine learning model is at least the chosen action or the modified action.
Clause 4. The method of clause 1, 2 or 3, wherein the agent is selected from the group consisting of: a contextual bandits reinforced learning machine learning model, and a set of encoded policies executable by a processor.
Clause 5. The method of any preceding clause, wherein: the state engine comprises software heuristics that, when executed, generates, from the data, a set of automated rules relating the behaviors of the users to outcomes of the behaviors for the users, the executable process comprises the set of automated rules, the agent comprises a machine learning model, an input of the machine learning model is the data, and an output of the machine learning model is at least the chosen action or the modified action.
Clause 6. The method of any preceding clause, wherein: the state engine comprises a plurality of additional machine learning models that generate, from the data, predictions of categorizations of the plurality of users, the state engine further comprises software heuristics that generates, from the predictions and the data, a set of automated rules relating the behaviors of the users to outcomes of the behaviors for the users, the executable process comprises the set of automated rules, the agent comprises a machine learning model, an input of the machine learning model is the data, and an output of the machine learning model is at least the chosen action or the modified action.
Clause 7. The method of any preceding clause, wherein: the problem comprises a first pre-defined financial behavior, the chosen action is a second pre-defined financial behavior, different than the first pre-defined financial behavior, the ongoing data comprises changes to measured financial data of the plurality of users, and the modified action is configured improve a probability that the plurality of users will at least adopt the second pre-defined financial behavior.
Clause 8. The method of any preceding clause, wherein: the problem comprises a first pre-defined academic behavior, the chosen action is a second pre-defined academic behavior, different than the first pre-defined academic behavior, the ongoing data comprises changes to measured academic data describing measured academic performance of the plurality of users, and the modified action is configured improve a probability that the plurality of users will at least adopt the second pre-defined academic behavior.
Clause 9. The method of any preceding clause, wherein the problem comprises a first pre-defined health-related behavior, the chosen action is a second pre-defined health-related behavior, different than the first pre-defined health-related behavior, the ongoing data comprises changes to measured medical data describing measured medical states of a plurality of users, and the modified action is configured improve a probability that the plurality of users will at least adopt the second pre-defined health-related behavior.
Clause 10. The method of any preceding clause, wherein: the parameter comprises a weight of a machine learning model that composes the agent, and the reward comprises a change to the weight.
Clause 11. The method of any preceding clause, further comprising: training, using the reward, a machine learning model that composes the agent.
Clause 12. The method of any preceding clause, wherein the reward is based on at least one of the group consisting of: click-through-rates of the plurality of users on the chosen action, a first measurement of a degree to which the plurality of users adopted the chosen action, a second measurement of a degree to which the plurality of users continue to have the problem after the plurality of users have viewed the chosen action, a third measurement that the data changes less than a first threshold amount after the plurality of users have viewed the chosen action, the third measurement indicating that a first number of the plurality of users continue to engage in a behavior, in the behaviors, pre-determined to be negative, and a fourth measurement that the data changes more than a second threshold amount after the plurality of users have viewed the chosen action, the fourth measurement indicating that a second number of the plurality of users have adopted a new behavior related to the chosen action, the new behavior pre-determined to be positive.
Clause 13. A system comprising: a processor; a data repository in communication with the processor, the data repository storing: data describing behaviors of a plurality of users operating in a computer environment, a problem of at least some of the plurality of users, a plurality of actions, a chosen action, from the plurality of actions, to alter the problem, a modified action, from the plurality of actions, a first electronic communication describing the chosen action, a second electronic communication describing the modified action, ongoing data, describing ongoing behaviors of the plurality of users, a reward configured to change a parameter, and an executable process; a state engine executable by the processor to: generate, from the data, an executable process, wherein the computer environment is external to the state engine, an agent executable by the processor to: execute the process by determining, from the data describing the behaviors of the plurality of users, a problem of at least some of the plurality of users, and selecting, based on the problem, a chosen action to alter the problem, transmit, at a first time, the first electronic communication to at least some of the plurality of users, monitor the ongoing data, generate the reward configured to change the parameter, wherein the parameter is associated with the agent, modify the agent to generate a modified agent, execute, by the modified agent, the executable process to select the modified action, and transmit, at a second time, the second electronic communication.
Clause 14. The system of clause 13, wherein the second time is selected by the agent to increase a probability that the at least some of the plurality of users adopt a new behavior suggested by the modified action in the second electronic communication.
Clause 15. The system of clause 13 or 14, wherein: the state engine comprises software heuristics that, when executed, generates, from the data, a set of automated rules relating the behaviors of the users to outcomes of the behaviors for the users, the executable process comprises the set of automated rules, the agent comprises a machine learning model, an input of the machine learning model is the data, and an output of the machine learning model is at least the chosen action or the modified action.
Clause 16. The system of clause 13, 14 or 15, wherein: the state engine comprises a plurality of additional machine learning models that generate, from the data, predictions of categorizations of the plurality of users, the state engine further comprises software heuristics that generates, from the predictions and the data, a set of automated rules relating the behaviors of the users to outcomes of the behaviors for the users, the executable process comprises the set of automated rules, the agent comprises a machine learning model, an input of the machine learning model is the data, and an output of the machine learning model is at least the chosen action or the modified action.
Clause 17. The system of any of clauses 13 to 16, wherein the agent is selected from the group consisting of: a contextual bandits reinforced learning machine learning model, and a set of encoded policies executable by a processor.
Clause 18. The system of any of clauses 13 to 17, further comprising: training, using the reward, a machine learning model that composes the agent.
Clause 19. The system of any of clauses 13 to 18, wherein the reward is based on at least one of the group consisting of: click-through-rates of the plurality of users on the chosen action, a first measurement of a degree to which the plurality of users adopted the chosen action, a second measurement of a degree to which the plurality of users continue to have the problem after the plurality of users have viewed the chosen action, a third measurement that the data changes less than a first threshold amount after the plurality of users have viewed the chosen action, the third measurement indicating that a first number of the plurality of users continue to engage in a behavior, in the behaviors, pre-determined to be negative, and a fourth measurement that the data changes more than a second threshold amount after the plurality of users have viewed the chosen action, the fourth measurement indicating that a second number of the plurality of users have adopted a new behavior related to the chosen action, the new behavior pre-determined to be positive.
Clause 20. A computer readable medium comprising computer readable program code which, when executed by a processor, implements a computer-implemented method comprising: generating, by a state engine from data describing behaviors of a plurality of users operating in a computer environment external to the state engine, an executable process; executing, by an agent, the executable process, by: determining, from the data describing the behaviors of the plurality of users, a problem of at least some of the plurality of users, and selecting, based on the problem, a chosen action to alter the problem; transmitting, at a first time, a first electronic communication describing the chosen action to the at least some of the plurality of users; monitoring ongoing data describing ongoing behaviors of the plurality of users; generating, based on the ongoing data, a reward, wherein the reward is configured to change a parameter of the agent; changing the parameter of the agent to generate a modified agent; executing, by the modified agent, the executable process to select a modified action; transmitting, at a second time, a second electronic communication describing the modified action.

While the present approaches and techniques have been described with respect to a limited number of embodiments, those skilled in the art, having benefit of this disclosure, will appreciate that other embodiments can be devised which do not depart from the scope as disclosed herein. Accordingly, the scope of the invention should be limited only by the attached claims.

## Claims

1. A method comprising:
generating, by a state engine from data describing behaviors of a plurality of users operating in a computer environment external to the state engine, an executable process;
executing, by an agent, the executable process, by:
determining, from the data describing the behaviors of the plurality of users, a problem of at least some of the plurality of users, and
selecting, based on the problem, a chosen action to alter the problem;
transmitting, at a first time, a first electronic communication describing the chosen action to the at least some of the plurality of users;
monitoring ongoing data describing ongoing behaviors of the plurality of users;
generating, based on the ongoing data, a reward, wherein the reward is configured to change a parameter of the agent;
changing the parameter of the agent to generate a modified agent;
executing, by the modified agent, the executable process to select a modified action;
transmitting, at a second time, a second electronic communication describing the modified action.

2. The method of claim 1, wherein the second time is selected by the agent to increase a probability that the at least some of the plurality of users adopt a new behavior suggested by the modified action in the second electronic communication.

3. The method of claim 1 or 2, wherein:
the agent comprises a machine learning model,
an input of the machine learning model comprises the data, and
an output of the machine learning model is at least the chosen action or the modified action.

4. The method of claim 1, 2 or 3, wherein the agent is selected from the group consisting of:
a contextual bandits reinforced learning machine learning model, and
a set of encoded policies executable by a processor.

5. The method of any preceding claim, wherein:
the state engine comprises software heuristics that, when executed, generates, from the data, a set of automated rules relating the behaviors of the users to outcomes of the behaviors for the users,
the executable process comprises the set of automated rules,
the agent comprises a machine learning model,
an input of the machine learning model is the data, and
an output of the machine learning model is at least the chosen action or the modified action.

6. The method of any preceding claim, wherein:
the state engine comprises a plurality of additional machine learning models that generate, from the data, predictions of categorizations of the plurality of users,
the state engine further comprises software heuristics that generates, from the predictions and the data, a set of automated rules relating the behaviors of the users to outcomes of the behaviors for the users,
the executable process comprises the set of automated rules,
the agent comprises a machine learning model,
an input of the machine learning model is the data, and
an output of the machine learning model is at least the chosen action or the modified action.

7. The method of any preceding claim, wherein:
the problem comprises a first pre-defined financial behavior,
the chosen action is a second pre-defined financial behavior, different than the first pre-defined financial behavior,
the ongoing data comprises changes to measured financial data of the plurality of users, and
the modified action is configured improve a probability that the plurality of users will at least adopt the second pre-defined financial behavior.

8. The method of any preceding claim, wherein:
the problem comprises a first pre-defined academic behavior,
the chosen action is a second pre-defined academic behavior, different than the first pre-defined academic behavior,
the ongoing data comprises changes to measured academic data describing measured academic performance of the plurality of users, and
the modified action is configured improve a probability that the plurality of users will at least adopt the second pre-defined academic behavior.

9. The method of any preceding claim, wherein
the problem comprises a first pre-defined health-related behavior,
the chosen action is a second pre-defined health-related behavior, different than the first pre-defined health-related behavior,
the ongoing data comprises changes to measured medical data describing measured medical states of a plurality of users, and
the modified action is configured improve a probability that the plurality of users will at least adopt the second pre-defined health-related behavior.

10. The method of any preceding claim, wherein:
the parameter comprises a weight of a machine learning model that composes the agent, and
the reward comprises a change to the weight.

11. The method of any preceding claim, further comprising:
training, using the reward, a machine learning model that composes the agent.

12. The method of any preceding claim, wherein the reward is based on at least one of the group consisting of:
click-through-rates of the plurality of users on the chosen action,
a first measurement of a degree to which the plurality of users adopted the chosen action,
a second measurement of a degree to which the plurality of users continue to have the problem after the plurality of users have viewed the chosen action,
a third measurement that the data changes less than a first threshold amount after the plurality of users have viewed the chosen action, the third measurement indicating that a first number of the plurality of users continue to engage in a behavior, in the behaviors, pre-determined to be negative, and
a fourth measurement that the data changes more than a second threshold amount after the plurality of users have viewed the chosen action, the fourth measurement indicating that a second number of the plurality of users have adopted a new behavior related to the chosen action, the new behavior pre-determined to be positive.

13. A system comprising:
a processor;
a data repository in communication with the processor, the data repository storing:
data describing behaviors of a plurality of users operating in a computer environment,
a problem of at least some of the plurality of users,
a plurality of actions,
a chosen action, from the plurality of actions, to alter the problem,
a modified action, from the plurality of actions,
a first electronic communication describing the chosen action,
a second electronic communication describing the modified action,
ongoing data, describing ongoing behaviors of the plurality of users,
a reward configured to change a parameter, and
an executable process;
a state engine executable by the processor to:
generate, from the data, an executable process, wherein the computer environment is external to the state engine,
an agent executable by the processor to:
execute the process by determining, from the data describing the behaviors of the plurality of users, a problem of at least some of the plurality of users, and selecting, based on the problem, a chosen action to alter the problem,
transmit, at a first time, the first electronic communication to at least some of the plurality of users,
monitor the ongoing data,
generate the reward configured to change the parameter, wherein the parameter is associated with the agent,
modify the agent to generate a modified agent,
execute, by the modified agent, the executable process to select the modified action, and
transmit, at a second time, the second electronic communication.

14. The system of claim 13, further configured to perform the method of any of claims 2 to 12.

15. A computer readable medium comprising computer readable program code which, when executed by a processor, cause the processor to implement the method of any of claims 1 to 12.
